# EUROPÄISCHE PATENTSCHRIFT

(11) **EP 4 520 301 B1**
(45) Veröffentlichungstag und Bekanntmachung des Hinweises auf die Patenterteilung: **20.05.2026**
(21) Anmeldenummer: 24198474.9
(22) Anmeldetag: 04.09.2024
(51) Int. Cl.: A61F 2/30, A61F 2/46, A61B 90/70, A61F 2/36

(54) **VORRICHTUNG ZUR REINIGUNG EINES KONUS EINES IMPLANTATS UND CHIRURGISCHES SYSTEM**
DEVICE FOR CLEANING A CONE OF AN IMPLANT AND SURGICAL SYSTEM
DISPOSITIF DE NETTOYAGE DE CÔNE D'IMPLANT ET SYSTÈME CHIRURGICAL

(30) Priorität: 11.09.2023 DE 202023105231 U
(43) Veröffentlichungstag der Anmeldung: 12.03.2025
(73) Patentinhaber: Peter Brehm Holding GmbH & Co. KG, 91085 Weisendorf (DE)
(72) Erfinder: Brehm, Peter, 91085 Weisendorf (DE); Kling, Janick, 91086 Aurachtal (DE); Schröder, Reinhard, 91325 Adelsdorf (DE)
(74) Vertreter: Grünecker Patent- und Rechtsanwälte PartG mbB

(56) Entgegenhaltungen:
- CN-U- 219 207 528
- US-A1- 2004 098 134
- US-A1- 2007 100 464
- US-A1- 2023 103 741
- STRYKER: "GMRS Proximal Femoral Surgical Protocol", 1 February 2011 (2011-02-01), pages 1 - 32, XP055753749, Retrieved from the Internet <URL:http://az621074.vo.msecnd.net/syk-mobile-content-cdn/global-content-system/SYKGCSDOC-2-39102/LpfVxU6BH5zQaSHYHR5sVtY_wEnvAQ/LSPK41.pdf> [retrieved on 20201125]

## Beschreibung

Die Erfindung ist in Anspruch 1 definiert und bezieht sich allgemein auf chirurgische Instrumente, insbesondere auf Vorrichtungen zur Reinigung eines Konus eines Implantats und auf chirurgische Systeme, die eine solche Vorrichtung umfassen.

Zum Ersatz eines natürlichen Gelenks eines Patienten, beispielsweise eines Hüft-, Schulter- oder Kniegelenks, das durch Degeneration, eine Krankheit oder eine Verletzung geschädigt ist, werden Endoprothesen verwendet. Endoprothesen sind Implantate, die in den Körper des Patienten implantiert werden und dort ein Gelenk ersetzen. Um eine bessere Anpassung an die Anatomie des Patienten zu ermöglichen, gibt es modulare Endoprothesen, die aus einzelnen Modulen aufgebaut sind. Die Module sind in verschiedenen Größen verfügbar. Je nach der Anatomie des Patienten können geeignete Module ausgewählt und miteinander kombiniert werden. Zum Verbinden der Module können Konusverbindungen verwendet werden, bei denen ein Konus an einem Modul in eine konusförmige Vertiefung eines anderen Moduls eingesetzt wird. Der Konus und die konusförmige Vertiefung weisen einen kleinen Öffnungswinkel auf, so dass nach dem Einsetzen des Konus in die konusförmige Vertiefung und/oder einer zusätzlichen Verspannung eine feste reibschlüssige Verbindung entsteht.

Modulare Hüftgelenksprothesen nach dem Stand der Technik, in denen Konusverbindungen verwendet werden, sind beispielsweise aus der DE 4 320 086 A1 und der EP 2 066 265 A2 bekannt.

Die US 2004/098134 A1 offenbart einen Stoff-/Gewebeschlauch, der dem Schutz und der Reinigung einer Konusverbindung dient. Weiterhin ist in CN 219207528 U ein motorisiertes Instrument in Form einer schalenförmigen Bürste zur Reinigung männlicher Implantatkonen offenbart.

Ein Problem, das bei modularen Endoprothesen, deren Module durch Konusverbindungen miteinander verbunden werden, auftritt, ist, dass durch Verunreinigungen zwischen dem Konus und der konusförmigen Vertiefung, in die der Konus eingesetzt wird, die Stabilität der Konusverbindung beeinträchtigt werden kann. Solche Verunreinigungen können während der Operation, bei der die Endoprothese dem Patienten implantiert wird, auftreten, beispielsweise durch Blut des Patienten. Der Konus muss deshalb vor dem Herstellen der Konusverbindung gereinigt werden, z.B. durch Abwischen mit einer Mullkompresse. Dabei kann es jedoch vorkommen, dass Verunreinigungen übersehen und nicht entfernt werden.

Es ist eine Aufgabe der Erfindung, eine Vorrichtung bereitzustellen, mit der der Konus eines Implantats während einer Operation zuverlässig von Verunreinigungen gereinigt werden kann.

Erfindungsgemäß wird die Aufgabe durch eine Vorrichtung zur Reinigung eines Konus eines Implantats gelöst, die ein Griffstück, eine innere Hülse und eine äußere Hülse umfasst. Das Griffstück hat ein proximales Ende und ein distales Ende, und weist eine axial durch das Griffstück verlaufende Durchgangsöffnung auf. Die Durchgangsöffnung weist an dem distalen Ende des Griffstücks einen ersten Abschnitt auf, der einen größeren Innendurchmesser als ein sich an den ersten Abschnitt anschließender zweiter Abschnitt der Durchgangsöffnung hat. Die innere Hülse ist in den zweiten Abschnitt der Durchgangsöffnung des Griffstücks einsetzbar und weist an einem ersten Ende einen Vorsprung auf. Dieser ist zur Anlage an einer Anlagefläche des Griffstücks, die sich am Übergang zwischen dem ersten und dem zweiten Abschnitt der Durchgangsöffnung des Griffstücks befindet, ausgebildet. Die äußere Hülse ist über einen distalen Abschnitt des Griffstücks am distalen Ende des Griffstücks schiebhar. Das Griffstück und die innere Hülse sind dafür ausgelegt, beim Einsetzen der inneren Hülse in den zweiten Abschnitt der Durchgangsöffnung des Griffstücks zwischen dem Vorsprung der inneren Hülse und der Anlagefläche des Griffstücks einen ersten Teil eines Textilstücks zu fixieren. Das Griffstück und die äußere Hülse sind dafür ausgelegt, beim Schieben der äußeren Hülse über den distalen Abschnitt des Griffstücks zwischen dem distalen Abschnitt des Griffstücks und der äußeren Hülse einen zweiten Teil des Textilstücks zu fixieren.

In der Vorrichtung kann ein Textilstück, das beispielsweise eine Mullkompresse sein kann, zum einen zwischen dem Vorsprung der inneren Hülse und der Anlagefläche am Übergang zwischen dem ersten und dem zweiten Abschnitt der Durchgangsöffnung des Griffstücks, und zum anderen zwischen dem distalen Abschnitt des Griffstücks und der äußeren Hülse fixiert werden, so dass es den ersten Abschnitt der Durchgangsöffnung bedeckt. Zur Reinigung des Konus des Implantats kann die Vorrichtung mit dem darin fixierten Textilstück auf den Konus aufgesetzt werden, so dass sich der Konus im ersten Abschnitt der Durchgangsöffnung des Griffstücks befindet. Durch Drehen der Vorrichtung wird dann das Textilstück über den Konus des Implantats bewegt, wodurch der Konus gereinigt wird. Da der Konus bei der Reinigung in den ersten Abschnitt der Durchgangsöffnung eingesetzt ist, kann der Konus ringsum gereinigt werden, wodurch das Risiko, dass Verunreinigungen am Konus verbleiben, verringert wird.

Geeigneterweise umfasst die Vorrichtung zusätzlich ein Montagewerkzeug. Das Montagewerkzeug weist an einem ersten Ende einen ersten Abschnitt, der in die innere Hülse einsetzbar ist, auf. An den ersten Abschnitt schließt sich ein zweiter Abschnitt an, der einen größeren Außendurchmesser als der erste Abschnitt hat und in den ersten Abschnitt der Durchgangsöffnung des Griffstücks einsetzbar ist. An den zweiten Abschnitt schließt sich ein dritter Abschnitt an, der einen größeren Außendurchmesser als der zweite Abschnitt hat und in die äußere Hülse einsetzbar ist.

Mit dem Montagewerkzeug können das Griffstück, die innere Hülse, die äußere Hülse und das Textilstück zusammengesetzt werden. Dazu wird die äußere Hülse über den dritten Abschnitt des Montagewerkzeugs geschoben und die innere Hülse wird auf den ersten Abschnitt des Montagewerkzeugs aufgesetzt. Dann wird das Textilstück mit einer Öffnung versehen und die innere Hülse wird durch die Öffnung des Textilstücks gesteckt. Danach wird das Griffstück auf das Montagewerkzeug aufgesetzt, wobei der zweite Abschnitt des Montagewerkzeugs in den ersten Abschnitt der Durchgangsöffnung des Griffstücks eingesetzt wird. Dabei wird das Textilstück so geformt, dass es an der Innenwand des ersten Abschnitts der Durchgangsöffnung anliegt. Beim Aufsetzen des Griffstücks auf das Montagewerkzeug wird das Textilstück am Rand seiner Öffnung zwischen dem Vorsprung der inneren Hülse und der Anlagefläche des Griffstücks fixiert. Anschließend wird die äußere Hülse nach oben über den distalen Abschnitt des Griffstücks geschoben, wodurch die Fixierung des zweiten Teils des Textilstücks zwischen dem distalen Abschnitt des Griffstücks und der Innenwand der inneren Hülse erfolgt.

Geeigneterweise weist das Montagewerkzeug an einem zweiten Ende eine Standfläche zum Stellen des Montagewerkzeugs auf eine Fläche auf. Dadurch kann die Montage einfacher durchgeführt werden.

Geeigneterweise umfasst die Vorrichtung zusätzlich einen Dorn mit einer Spitze zum Durchstechen des Textilstücks. Der Dorn weist an einem von der Spitze abgewandten Ende einen Halteabschnitt auf, der an dem von ihrem ersten Ende abgewandten zweiten Ende der inneren Hülse in die innere Hülse einsetzbar ist. Der Dorn kann nach dem Aufsetzen der inneren Hülse auf den ersten Abschnitt des Montagewerkzeugs auf die innere Hülse aufgesetzt werden, wobei der Halteabschnitt des Dorns in die innere Hülse eingesetzt wird. Dann wird das Textilstück zum Erzeugen der Öffnung des Textilstücks über den Dorn nach unten gezogen, bis es auf dem Vorsprung der inneren Hülse aufliegt, und der Dorn wird wieder entfernt.

Geeigneterweise hat ein Teil des Dorns einen größeren Außendurchmesser als der zweite Abschnitt der Durchgangsöffnung des Griffstücks, so dass der Dorn nicht durch den zweiten Abschnitt der Durchgangsöffnung passt. Dadurch wird verhindert, dass die innere Hülse in den zweiten Abschnitt der Durchgangsöffnung des Griffstücks eingesetzt wird, während sich der Dorn noch auf der inneren Hülse befindet. Dadurch kann eine falsche Montage der Vorrichtung verhindert werden.

Geeigneterweise weist die innere Hülse einen oder mehrere, insbesondere drei, Schlitze auf.

Geeigneterweise hat die innere Hülse in dem Bereich, in dem die Schlitze angeordnet sind, eine nach außen gewölbte Wand, die eine Elastizität aufweist, um die in den zweiten Abschnitt der Durchgangsöffnung des Griffstücks eingesetzte innere Hülse an der Innenwand des zweiten Abschnitts der Durchgangsöffnung des Griffstücks festzuhalten. Durch die elastische, nach außen gewölbte Wand kann die innere Hülse im zweiten Abschnitt der Durchgangsöffnung des Griffstücks festgeklemmt werden, wodurch das Risiko, dass die innere Hülse nach unten aus dem Griffstück herausrutscht, verringert wird. Durch die Schlitze wird ein Bewegungsspielraum für die Abschnitte der nach außen gewölbten Wand zwischen den Schlitzen bereitgestellt, so dass sie sich leichter elastisch verformen können.

Geeigneterweise weist der distale Abschnitt des Griffstücks einen ersten Bereich und einen zweiten Bereich auf, die jeweils einen größeren Außendurchmesser als ein zwischen dem ersten und dem zweiten Bereich angeordneter dritten Bereich haben. Der erste und der zweite Bereich des Griffstücks sind an den Enden der äußeren Hülse angeordnet, wenn die äußere Hülse über den distalen Abschnitt des Griffstücks geschoben ist. Dadurch kann das Textilstück zwischen dem ersten und dem zweiten Bereich des distalen Abschnitts des Griffstücks und der Innenwand der Hülse eingeklemmt werden, so dass es an beiden Enden der äußeren Hülse fixiert ist. Durch den kleineren Außendurchmesser des dritten Bereichs zwischen dem ersten und dem zweiten Bereich des distalen Abschnitts des Griffstücks ist zwischen dem dritten Bereich und der Innenwand der äußeren Hülse ein Raum vorhanden, der Teile des Textilstücks aufnehmen kann.

Geeigneterweise weist der distale Abschnitt des Griffstücks in dem dritten Bereich einen oder mehrere, insbesondere zwei, Schlitze auf, die gegenüber von dem zweiten Ende der inneren Hülse und/oder dem einen oder den mehreren Schlitzen der inneren Hülse angeordnet sind, wenn die innere Hülse in den zweiten Abschnitt der Durchgangsöffnung des Griffstücks eingesetzt ist. Dadurch kann die innere Hülse mit Hilfe eines Werkzeugs, das durch die Schlitze im distalen Abschnitt des Griffstücks eingeführt wird, herausgeschoben werden, falls sich die innere Hülse im Griffstück unbeabsichtigt festklemmt.

Geeigneterweise ist der zweite Bereich des distalen Abschnitts des Griffstücks weiter vom distalen Ende des Griffstücks entfernt als der erste Bereich. Der Außendurchmesser des zweiten Bereichs des distalen Abschnitts des Griffstücks und ein Innendurchmesser der äußeren Hülse sind so ausgelegt, dass der zweite Bereich des distalen Abschnitts des Griffstücks einen Anschlag für die äußere Hülse bildet, wenn sich das Textilstück zwischen dem zweiten Bereich des distalen Abschnitts des Griffstücks und der äußeren Hülse befindet. Wenn sich das Textilstück nicht zwischen dem zweiten Bereich des distalen Abschnitts des Griffstücks und der äußeren Hülse befindet, ist die äußere Hülse über den zweiten Bereich des distalen Abschnitts des Griffstücks schiebbar.

Durch den Anschlag für die äußere Hülse kann der zweite Teil des Textilstücks sicherer zwischen dem distalen Abschnitt des Griffstücks und der äußeren Hülse fixiert werden. Da der Anschlag nur vorhanden ist, wenn sich das Textilstück zwischen dem zweiten Bereich des distalen Abschnitts des Griffstücks und der äußeren Hülse befindet, fällt es auf, wenn das Textilstück nicht richtig zwischen dem zweiten Bereich des distalen Abschnitts des Griffstücks und der äußeren Hülse angeordnet ist, da dann die äußere Hülse weiter nach oben geschoben werden kann. Dadurch wird die Sicherheit der Vorrichtung verbessert.

Geeigneterweise weist das Griffstück an dem proximalen Ende mehrere axial verlaufende Rillen an der Außenseite des Griffstücks auf. Dadurch kann das Griffstück beim Drehen der Vorrichtung zur Reinigung des Konus besser gehalten werden.

Geeigneterweise hat die Durchgangsöffnung des Griffstücks am proximalen Ende des Griffstücks einen Innendurchmesser, der kleiner als ein Außendurchmesser der inneren Hülse und kleiner als der Innendurchmesser des zweiten Abschnitts der Durchgangsöffnung am distalen Ende des Griffstücks ist. Dadurch kann die innere Hülse am proximalen Ende des Griffstücks nicht in die Durchgangsöffnung eingesetzt werden, wodurch verhindert wird, dass das Griffstück falsch herum auf die innere Hülse aufgesetzt wird.

Geeigneterweise weist der erste Abschnitt der Durchgangsöffnung an seiner Wandfläche eine Riffelung auf. Dadurch kann ein Verdrehen des Textilstücks während der Reinigung des Konus verhindert werden.

Geeigneterweise ist das Textilstück eine Mullkompresse. Mullkompressen sind dafür geeignet, Blut des Patienten und andere Verunreinigungen aufzusaugen, so dass Verunreinigungen zuverlässig vom Konus des Implantats entfernt werden können.

Erfindungsgemäß wird die oben genannte Aufgabe außerdem durch eine Vorrichtung zur Reinigung eines Konus eines Implantats, die ein Griffstück, eine innere Hülse, eine äußere Hülse und ein Textilstück umfasst, gelöst. Das Griffstück hat ein proximales Ende und ein distales Ende. Durch das Griffstück verläuft axial eine Durchgangsöffnung. Die Durchgangsöffnung weist an dem distalen Ende des Griffstücks einen ersten Abschnitt auf, der einen größeren Innendurchmesser als ein sich an den ersten Abschnitt anschließender zweiter Abschnitt der Durchgangsöffnung hat. Am Übergang zwischen dem ersten und dem zweiten Abschnitt der Durchgangsöffnung des Griffstücks befindet sich eine Anlagefläche. Die innere Hülse ist in den zweiten Abschnitt der Durchgangsöffnung des Griffstücks eingesetzt und weist an einem ersten Ende einen Vorsprung auf. Die äußere Hülse umschließt einen distalen Abschnitt des Griffstücks am distalen Ende des Griffstücks. Das Textilstück weist eine Öffnung auf, durch die die innere Hülse geführt ist. Zwischen dem Vorsprung der inneren Hülse und der Anlagefläche des Griffstücks ist ein erster Teil des Textilstücks fixiert. Außerdem ist ein zweiter Teil des Textilstücks zwischen dem distalen Abschnitt des Griffstücks und der äußeren Hülse fixiert.

Ein erfindungsgemäßes chirurgisches System umfasst eine Vorrichtung zur Reinigung eines Konus eines Implantats wie oben beschrieben, und ein Implantat, das einen Konus aufweist. Der Konus des Implantats und der erste Abschnitt der Durchgangsöffnung des Griffstücks der Vorrichtung zur Reinigung eines Konus eines Implantats haben zueinander korrespondierende Formen.

Da der erste Abschnitt der Durchgangsöffnung des Griffstücks und der Konus zueinander korrespondierende Formen haben, wird das Textilstück durch die Innenwand des ersten Abschnitts der Durchgangsöffnung des Griffstücks bei der Reinigung des Konus gegen die gesamte Fläche des Konus gedrückt, so dass die gesamte Fläche des Konus zuverlässig gereinigt werden kann.

Geeigneterweise ist das Implantat ein Verankerungsschaft einer Endoprothese, insbesondere einer Hüft-, Schulter- oder Knieprothese.

Geeigneterweise weist der Konus des Implantats eine Öffnung mit einem Innengewinde auf. Eine Achse der Öffnung des Konus des Implantats liegt auf einer Kegelachse des Konus. Das System umfasst zusätzlich einen Führungsstab, der in die Öffnung des Konus des Implantats einschraubbar ist. Der Führungsstab ist durch die Durchgangsöffnung des Griffstücks und die in den zweiten Abschnitt der Durchgangsöffnung des Griffstücks eingesetzte innere Hülse der Vorrichtung zur Reinigung eines Konus einer Endoprothese führbar.

Durch den Führungsstab wird die Vorrichtung geführt, während sie zur Reinigung des Konus des Implantats auf diesen aufgesetzt wird. Dadurch kann ein schiefes Aufsetzen der Vorrichtung auf dem Konus verhindert werden. Nach der Reinigung des Konus kann der Führungsstab außerdem zum Aufsetzen eines Moduls mit einer konusförmigen Vertiefung auf den Konus und zum Verspannen der Konusverbindung verwendet werden.

Im Folgenden werden Ausführungsformen der Erfindung mit Bezug auf die Figuren beschrieben.
Fig. 1 zeigt eine schematische Querschnittsansicht einer Vorrichtung zur Reinigung eines Konus eines Implantats gemäß einer Ausführungsform der Erfindung.
Fig. 2 zeigt eine Ansicht eines Griffstücks der Vorrichtung zur Reinigung eines Konus eines Implantats.
Fig. 3 zeigt eine schematische Ansicht einer inneren Hülse der Vorrichtung zur Reinigung eines Konus eines Implantats.
Fig. 4 zeigt eine schematische Ansicht einer äußeren Hülse der Vorrichtung zur Reinigung eines Konus eines Implantats.
Fig. 5 zeigt eine schematische Ansicht eines Montagewerkzeugs der Vorrichtung zur Reinigung eines Konus eines Implantats.
Fig. 6 zeigt eine schematische Ansicht eines Dorns der Vorrichtung zur Reinigung eines Konus eines Implantats.
Fig. 7 zeigt eine schematische Ansicht eines Implantats und eines mit dem Implantat verbundenen Führungsstabs.

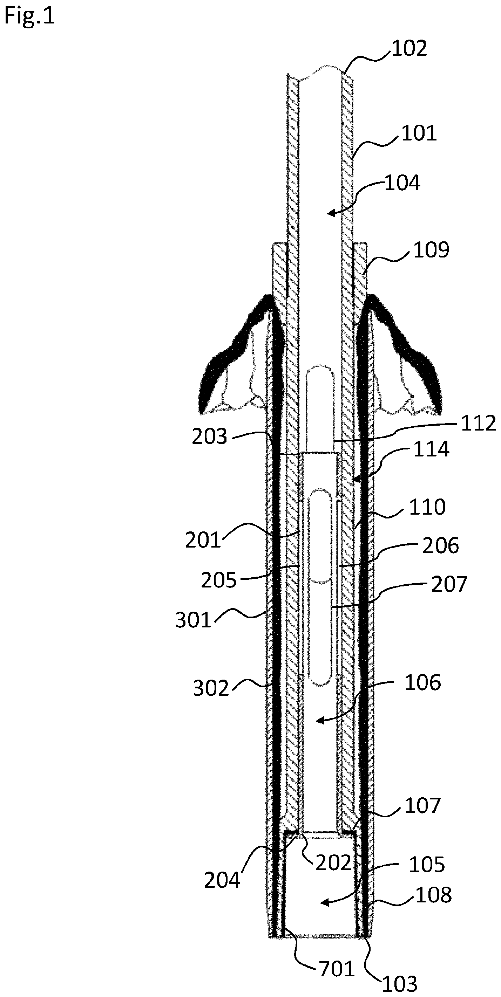

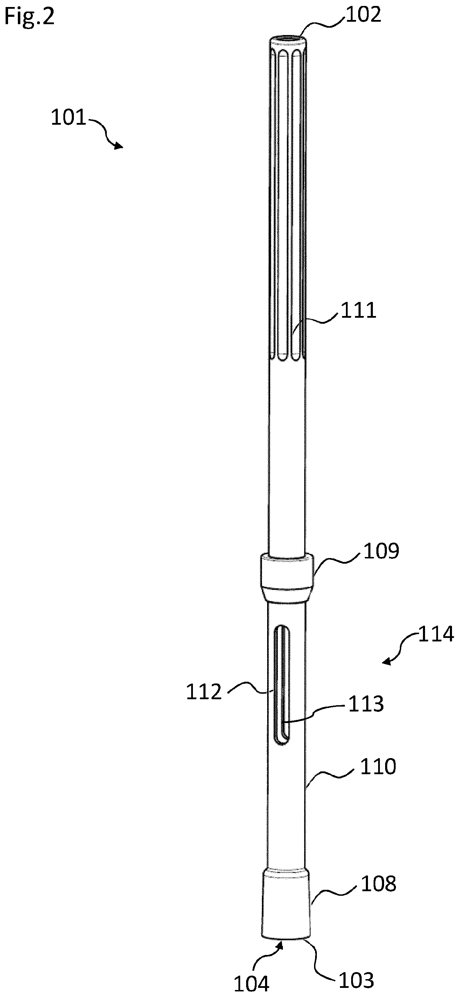

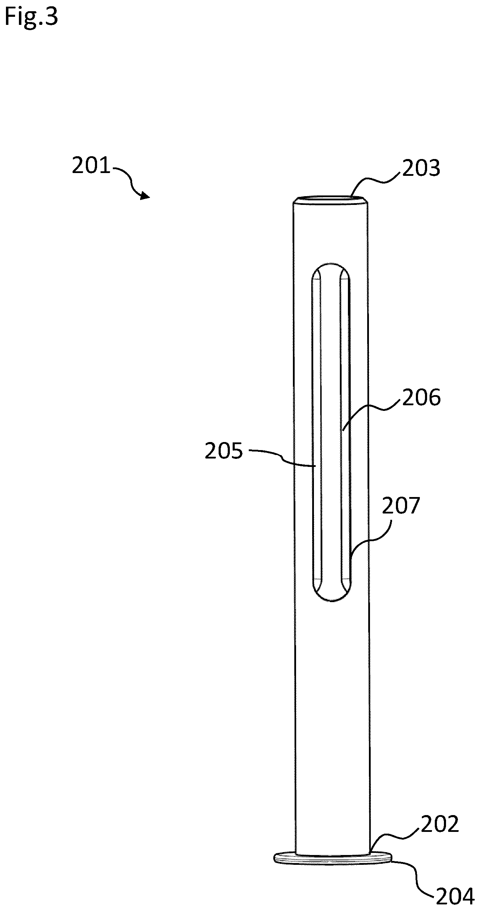

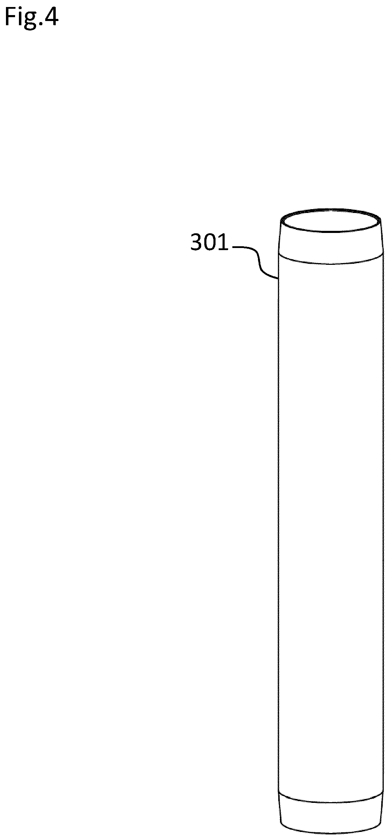

Fig. 1 zeigt eine schematische Querschnittsansicht einer Vorrichtung zur Reinigung eines Konus eines Implantats gemäß einer Ausführungsform der Erfindung. Die Vorrichtung umfasst ein Griffstück 101, eine innere Hülse 201 und eine äußere Hülse 301. Schematische Ansichten des Griffstücks 101, der inneren Hülse 201 und der äußeren Hülse 301 sind in den Fig. 2, 3 und 4 gezeigt.

Die Fig. 1 zeigt das Griffstück 101, die innere Hülse 201 und die äußere Hülse 301 in der Anordnung, in der sie sich befinden, wenn die Vorrichtung zur Reinigung des Konus eines Implantats verwendet wird. Bei der Verwendung der Vorrichtung wird vom Griffstück 101, der inneren Hülse 201 und der äußeren Hülse 301 ein Textilstück 701 fixiert, das bei der Reinigung des Konus über den Konus bewegt wird, um Verunreinigungen vom Konus zu entfernen. Das Textilstück 701 kann eine Mullkompresse sein, beispielsweise eine 16-fache oder 12-fache Mullkompresse, die zusammengefaltet eine Größe von 10 x 10 cm hat und vor dem Einsetzen in die Vorrichtung teilweise auseinandergefaltet wird.

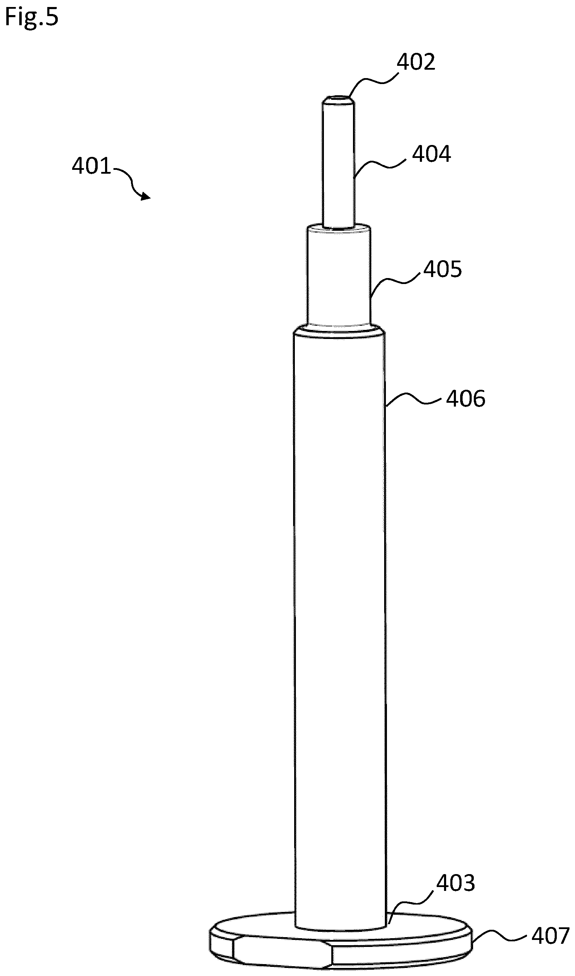

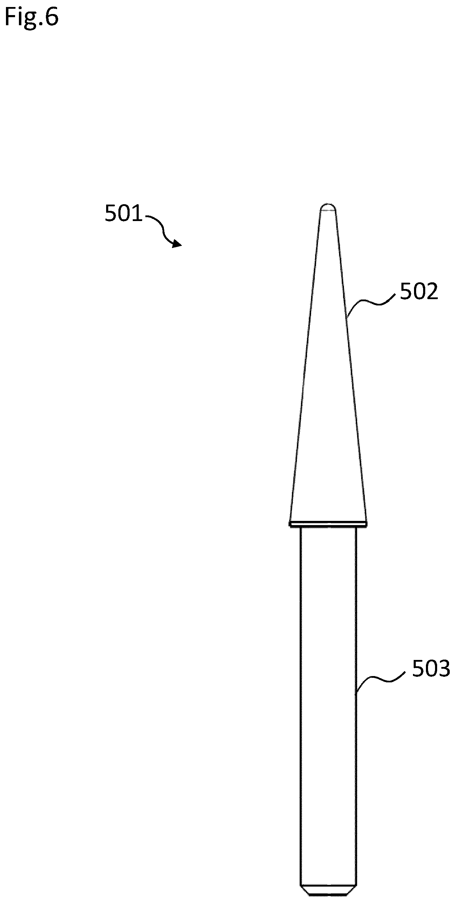

Die Vorrichtung gemäß der Ausführungsform umfasst außerdem ein Montagewerkzeug 401, das in Fig. 5 dargestellt ist, und einen Dorn 501, der in Fig. 6 dargestellt ist.

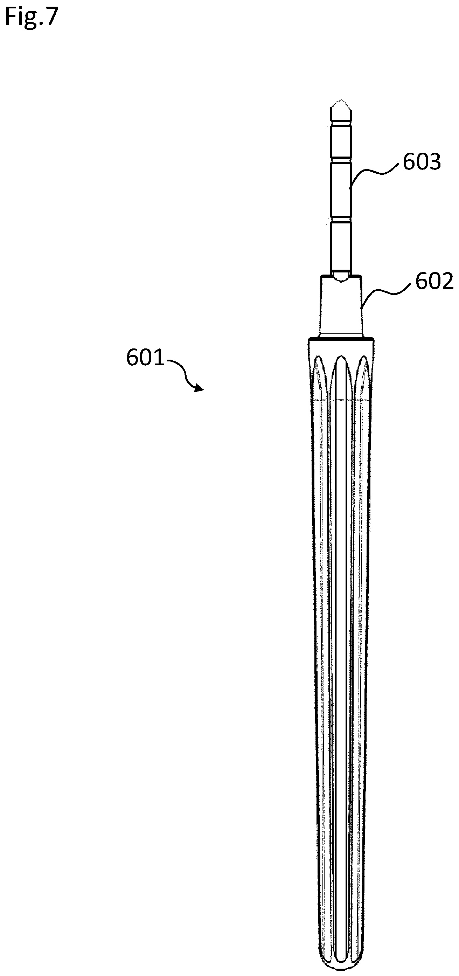

Ein Beispiel eines Implantats 601 mit einem Konus 602, der mit der Vorrichtung gemäß der Ausführungsform gereinigt werden kann, ist in Fig. 7 dargestellt. Das in Fig. 7 dargestellte Implantat 601 ist ein Verankerungsschaft. Der Verankerungsschaft 601 kann Teil einer Endoprothese, beispielsweise eines künstlichen Hüftgelenks, sein. Der Verankerungsschaft 601 wird in einen Röhrenknochen, beispielweise in das Femur, des Patienten eingesetzt. Auf dem Konus 602 des Verankerungsschafts 601 kann ein Modul mit einer zur Form des Konus 602 korrespondierenden konusförmigen Vertiefung aufgesetzt werden, das wiederum mit einer Gelenkkugel verbunden wird, die in z.B. eine Azetabularkomponente des künstlichen Hüftgelenks eingesetzt wird. Der Konus 602 kann eine Öffnung mit einem Innengewinde aufweisen, wobei eine Achse der Öffnung des Konus 602 auf einer Kegelachse des Konus 602 liegt. In diese Öffnung kann ein Führungsstab 603 eingeschraubt werden, der beim Zusammenbau der Endoprothese sowie, wie im Folgenden beschrieben wird, bei der Reinigung des Konus 602 verwendet werden kann. Verankerungsschäfte ähnlich dem Verankerungsschaft 601 können in Hüft-, Schulter- und Kniegelenkprothesen verwendet werden.

Das Griffstück 101 der Vorrichtung hat ein proximales Ende 102 und ein distales Ende 103. Bei der Verwendung der Vorrichtung zum Reinigen eines Konus eines Implantats kann das Griffstück 101 in der Nähe des proximalen Endes 102 gehalten werden. Für einen besseren Halt kann das Griffstück 101 an seiner Außenseite mehrere axial verlaufende Rillen 111 aufweisen.

Das Griffstück 101 weist eine axial durch das Griffstück 101 verlaufende Durchgangsöffnung 104 auf. An dem distalen Ende 103 des Griffstücks 101 befindet sich ein erster Abschnitt der Durchgangsöffnung 104. An den ersten Abschnitt 105 schließt sich ein zweiter Abschnitt 106 der Durchgangsöffnung 104 an. Der erste Abschnitt 105 der Durchgangsöffnung 104 hat einen größeren Innendurchmesser als der zweite Abschnitt 106. Am Übergang zwischen dem ersten Abschnitt 105 und dem zweiten Abschnitt 106 befindet sich eine Anlagefläche 107. Die Anlagefläche 107 kann eine ringförmige Fläche sein, die senkrecht zur axialen Richtung (in Fig. 1 vertikal) ist.

Die Innenwand des ersten Abschnitts 105 der Durchgangsöffnung 104 kann eine Form haben, die zur Form des Konus 602 des Implantats 601 korrespondiert. Beispielsweise kann die Innenwand des ersten Abschnitts 105 der Durchgangsöffnung eine konische Form mit einem dem Öffnungswinkel des Konus 602 entsprechenden Öffnungswinkel haben, so dass das Textilstück 701 auf der ganzen Fläche des Konus 602 gleichmäßig gegen den Konus 602 gedrückt wird, wenn das Griffstück 101 auf den Konus 602 aufgesetzt wird. Alternativ kann der erste Abschnitt 105 der Durchgangsöffnung 104 auch eine zylindrische Form haben. Da der Konus 602 des Implantats 601 einen relativ kleinen Öffnungswinkel hat, kann wegen der Elastizität des Textilstücks 701 auch in diesem Fall das Textilstück 701 gegen die gesamte Fläche des Konus 602 gedrückt werden.

Eine Wandfläche der Innenwand des ersten Abschnitts 105 der Durchgangsöffnung 104 kann eine Riffelung aufweisen. Dadurch kann eine Verdrehung des Textilstücks 701 während der Verwendung der Vorrichtung verhindert oder zumindest reduziert werden.

An das distale Ende 103 des Griffstücks 101 schließt sich ein distaler Abschnitt 114 des Griffstücks 101 an. Der distale Abschnitt 114 des Griffstücks 101 umfasst einen ersten Bereich 108, der sich unmittelbar an das distale Ende 103 anschließt, einen zweiten Bereich 109, der weiter vom distalen Ende 103 entfernt ist als der erste Bereich 108, und einen dritten Bereich 110, der sich zwischen dem ersten Bereich 108 und dem zweiten Bereich 109 befindet. Der erste Bereich 108 und der zweite Bereich 109 haben einen größeren Außendurchmesser als der dritte Bereich 110. Der Außendurchmesser des zweiten Bereichs 109 kann etwas größer als der Außendurchmesser des ersten Bereichs 108 sein.

Die innere Hülse 201 hat ein erstes Ende 202 und ein zweites Ende 203. Am ersten Ende 202 der inneren Hülse 201 weist diese einen Vorsprung 204 auf. Die innere Hülse 201 ist, wie in Fig. 1 dargestellt, in den zweiten Abschnitt 106 der Durchgangsöffnung 104 des Griffstücks einsetzbar, so dass sich das zweite Ende 203 der inneren Hülse im Inneren des zweiten Abschnitts 106 der Durchgangsöffnung 104 befindet. Der Vorsprung 204 hat einen Außendurchmesser, der größer ist als der Innendurchmesser des zweiten Abschnitts 106 der Durchgangsöffnung 104 in dem Bereich, der sich an den ersten Abschnitt 105 der Durchgangsöffnung 104 anschließt, so dass er beim Einsetzen der inneren Hülse 201 in den zweiten Abschnitt 106 der Durchgangsöffnung 104 an der Anlagefläche 107 des Griffstücks 101 anliegt und verhindert, dass die innere Hülse 201 zu weit in den zweiten Abschnitt 106 der Durchgangsöffnung geschoben wird.

Wenn die Vorrichtung zur Reinigung eines Konus eines Implantats verwendet wird, befindet sich zwischen dem Vorsprung 204 der inneren Hülse und der Anlagefläche 107 des Griffstücks 101 ein Teil des Textilstücks 701, das dadurch von dem Vorsprung 204 der inneren Hülse 201 und der Anlagefläche 107 fixiert wird. Dabei erstreckt sich die innere Hülse 201 durch eine Öffnung des Textilstücks 701, so wie dies in Fig. 1 dargestellt ist.

Die innere Hülse 201 kann Schlitze 205, 206, 207 aufweisen, beispielsweise drei Schlitze. Die drei Schlitze 205, 206, 207 können jeweils um einen Winkel von 120° versetzt am Umfang der inneren Hülse 201 angeordnet sein. In dem Bereich, in dem die Schlitze 205, 206, 207 der inneren Hülse 201 angeordnet sind, kann die Wand der inneren Hülse 201 leicht nach außen gewölbt sein. Wenn die innere Hülse 201 in den zweiten Abschnitt 106 der Durchgangsöffnung 104 des Griffstücks 101 eingesetzt wird, wird die nach außen gewölbte Wand elastisch verformt, so dass sie auf die Innenwand des zweiten Abschnitts 106 der Durchgangsöffnung 104 drückt. Dadurch entsteht Reibung zwischen der inneren Hülse 201 und der Innenwand des zweiten Abschnitts 106 der Durchgangsöffnung 104, durch die die innere Hülse 201 an der Innenwand des zweiten Abschnitts 106 der Durchgangsöffnung 104 festgehalten wird. Durch die Schlitze 205, 206, 207 der inneren Hülse kann sich die Wand der inneren Hülse leichter elastisch verformen.

Das Griffstück 101 kann in dem dritten Bereich 110 des distalen Abschnitts 114 Schlitze 112, 113 aufweisen, beispielsweise zwei Schlitze. Die beiden Schlitze 112, 113 könne um einen Winkel von 180° versetzt am Umfang des Griffstücks 101 angeordnet sein. Wenn die innere Hülse 201 in den zweiten Abschnitt 106 der Durchgangsöffnung 104 des Griffstücks 101 eingesetzt ist, liegen die Schlitze 112, 113 des Griffstücks 101 gegenüber von den Schlitzen 205, 206, 207 der inneren Hülse 201 und/oder dem zweiten Ende 203 der inneren Hülse 201. Sollte sich die innere Hülse 201 unbeabsichtigt im Inneren des zweiten Abschnitts 106 der Durchgangsöffnung 104 des Griffstücks 101 festklemmen, kann die innere Hülse 201 mit Hilfe eines Werkzeugs, das durch einen der Schlitze 112, 113 des Griffstücks geführt wird, gelöst werden, indem mit dem Werkzeug am zweiten Ende 203 der inneren Hülse 201 oder am Ende von einem der Schlitze 205, 206, 207 der inneren Hülse 201 geschoben wird.

Die Durchgangsöffnung 104 des Griffstücks 101 kann am proximalen Ende 102 des Griffstücks 101 einen Innendurchmesser haben, der kleiner als der Außendurchmesser der inneren Hülse 201 und kleiner als der Innendurchmesser des zweiten Abschnitts 106 der Durchgangsöffnung 104 in der Nähe des distalen Endes 103 des Griffstücks 101 ist. Beispielsweise kann sich der zweite Abschnitt 106 der Durchgangsöffnung 104 in einem Bereich, der näher am proximalen Ende 102 liegt als der Bereich, in dem sich die in den zweiten Abschnitt 106 der Durchgangsöffnung 104 eingesetzte innere Hülse 201 befindet, verjüngen. So kann die innere Hülse 201 nicht am proximalen Ende des Griffstücks 102 in die Durchgangsöffnung 104 eingesetzt werden, wodurch ein falsches Einsetzen der inneren Hülse 201 verhindert wird.

Die äußere Hülse 301 kann, wie in Fig. 1 dargestellt, über den distalen Abschnitt 114 des Griffstücks 101 geschoben werden. Der erste Bereich 108 und der zweite Bereich 109 des Griffstücks 101 sind dann an den Enden der äußeren Hülse 301 angeordnet. Ein Teil des zweiten Bereichs 109 kann dann, wie in Fig. 1 gezeigt, über das Ende der äußeren Hülse 301 hinausragen. Bei der Verwendung der Vorrichtung zur Reinigung des Konus eines Implantats befindet sich ein Teil des Textilstücks 701 zwischen dem distalen Abschnitt 114 des Griffstücks 101 und der Innenwand 302 der äußeren Hülse 301. Im ersten Bereich 108 und im zweiten Bereich 109 des distalen Abschnitts 114 des Griffstücks 101 wird dabei das Textilstück 701 zwischen dem Griffstück 101 und der Innenwand 302 der äußeren Hülse 301 eingeklemmt, so dass es fixiert ist.

Der Innendurchmesser der äußeren Hülse 301 ist etwas größer als der Außendurchmesser des ersten Bereichs 108 und des zweiten Bereichs 109 des distalen Abschnitts 114 des Griffstücks 101, so dass das Textilstück 701 zwischen dem Griffstück 101 und der Innenwand der äußeren Hülse 301 Platz hat. Der Außendurchmesser des zweiten Bereichs 109 des distalen Abschnitts 114 des Griffstücks 101 und der Innendurchmesser der äußeren Hülse 301 können so ausgelegt sein, dass der zweite Bereich 109 des distalen Abschnitts 114 des Griffstücks 101 einen Anschlag für die äußere Hülse 301 bildet, wenn sich das Textilstück 701 zwischen dem zweiten Bereich 109 des distalen Abschnitts 114 des Griffstücks 101 und der äußeren Hülse 301 befindet. Die äußere Hülse 301 kann dann nicht wesentlich weiter als bis zu der in Fig. 1 dargestellten Position zum proximalen Ende 102 des Griffstücks 101 hin geschoben werden. Wenn das Textilstück 701 nicht richtig sitzt, so dass es sich nicht oder nicht in ausreichendem Maß zwischen dem zweiten Bereich 109 des distalen Abschnitts 114 des Griffstücks 101 und der Innenwand 302 der äußeren Hülse 301 befindet, kann die äußere Hülse 301 über die in Fig. 1 dargestellte Position hinaus zum proximalen Ende 102 des Griffstücks 101 hin geschoben werden. So kann erkannt werden, dass das Textilstück 701 nicht richtig in die Vorrichtung eingelegt ist. Dadurch wird die Sicherheit der Benutzung der Vorrichtung verbessert.

Das Textilstück 701 kann somit sowohl zwischen dem Vorsprung 204 der inneren Hülse 201 und der Anlagefläche 107 des Griffstücks 101, als auch zwischen den Bereichen 108, 109 des distalen Abschnitts 114 des Griffstücks 101 und der äußeren Hülse 301 fixiert werden, so dass es an der Innenwand des zweiten Abschnitts 106 der Durchgangsöffnung 104 anliegt.

Die Montagevorrichtung 401 hat ein erstes Ende 402 und ein zweites Ende 403. Am ersten Ende 402 befindet sich ein erster Abschnitt 404, dessen Außendurchmesser so ausgelegt ist, dass er in die innere Hülse 201 einsetzbar ist. An den ersten Abschnitt 404 schließt sich ein zweiter Abschnitt 405 des Montagewerkzeugs 401 an, der einen größeren Außendurchmesser als der erste Abschnitt 404 hat und in den ersten Abschnitt 105 der Durchgangsöffnung 104 des Griffstücks einsetzbar ist. An den zweiten Abschnitt 405 schließt sich ein dritter Abschnitt 406 an, der einen größeren Außendurchmesser als der zweite Abschnitt 405 hat und in die äußere Hülse 301 einsetzbar ist. Am zweiten Ende 403 des Montagewerkzeugs 401 befindet sich eine Standfläche 407, mit der das Montagewerkzeug 401 während der Montage der Vorrichtung auf eine Fläche gestellt werden kann.

Der Dorn 501 hat eine Spitze 502 und einen Halteabschnitt 503. Der Halteabschnitt 503 hat einen kleineren Außendurchmesser als der Innendurchmesser der inneren Hülse 201, so dass er am zweiten Ende 203 der inneren Hülse 201 in diese eingesetzt werden kann.

Die Spitze 502 des Dorns 501 kann eine Kegelform haben. Ein Teil des Dorns 501, beispielsweise in dem Bereich der Spitze 502, der sich unmittelbar an den Halteabschnitt 503 anschließt, hat einen größeren Außendurchmesser als der zweite Abschnitt 106 der Durchgangsöffnung 104 des Griffstücks 101, so dass der Dorn 501 nicht durch den zweiten Abschnitt 106 der Durchgangsöffnung 104 passt. Dadurch wird verhindert, dass der Dorn 501 versehentlich zusammen mit der inneren Hülse 201 in den zweiten Abschnitt 106 der Durchgangsöffnung 104 des Griffstücks 101 eingesetzt wird. Dadurch wird die Sicherheit der Benutzung der Vorrichtung verbessert.

Zum Zusammenbau der Vorrichtung wird zunächst die Standfläche 407 des Montagewerkzeugs 401 auf eine ebene Fläche gestellt und die äußere Hülse 301 auf das Montagewerkzeug 401 geschoben, so dass sich der dritte Abschnitt 406 des Montagewerkzeugs 401 in der äußeren Hülse 301 befindet. Anschließend wird die innere Hülse 201 auf den ersten Abschnitt 404 des Montagewerkzeug 401 aufgesetzt, und der Halteabschnitt 503 des Dorns 501 am zweiten Ende 203 der inneren Hülse 201 in diese eingesetzt. Dann wird das Textilstück 701 so auf die Spitze 502 des Dorns 501 aufgesetzt, dass diese sich ungefähr in der Mitte des Textilstücks 701 befindet, und das Textilstück 701 wird nach untern geschoben, bis es auf dem Vorsprung 204 der inneren Hülse 201 aufliegt. Dabei wird es von der Spitze 502 des Dorns 501 durchstochen, so dass das Textilstück 701 eine Öffnung erhält, deren Rand auf dem Vorsprung 204 der inneren Hülse 201 aufliegt. Dann wird der Dorn 501 entfernt und das Griffstück 101 über die innere Hülse 201 geschoben, bis der Vorsprung 204 der inneren Hülse, auf der das Textilstück 701 aufliegt, an der Anlagefläche 107 des Griffstücks 101 anliegt. Danach wird die äußere Hülse 301 nach oben geschoben, bis sie sich in der in Fig. 1 dargestellten Position befindet.

Anschließend kann die Vorrichtung zur Reinigung des Konus 602 des Implantats 601 verwendet werden. Dazu wird sie mit dem distalen Ende 103 des Griffstücks 101 voran auf den Konus 602 des Implantats 601 aufgesetzt. Der Führungsstab 603, der wie in Fig. 7 gezeigt mit dem Implantat 601 verbunden ist, wird dabei in die Durchgangsöffnung 104 des Griffstücks 101 sowie in die innere Hülse 201 eingesetzt. Dann wird die Vorrichtung gedreht, so dass der Konus 602 von dem Teil des Textilstücks 701, das an der Innenwand des ersten Abschnitts 105 der Durchgangsöffnung 104 des Griffstücks 101 anliegt, von Verunreinigungen befreit wird. Danach kann die Vorrichtung von dem Konus 602 des Implantats und dem Führungsstab 603 abgezogen werden, und auf den gereinigten Konus 602 kann ein Modul der Endoprothese mit einer zum Konus 602 passenden konusförmigen Vertiefung aufgesetzt werden, um eine Konusverbindung herzustellen.

## Patentansprüche

1. Vorrichtung zur Reinigung eines Konus (602) eines Implantats (601), umfassend:
ein Griffstück (101) mit einem proximalen Ende (102), einem distalen Ende (103) und einer axial durch das Griffstück (101) verlaufenden Durchgangsöffnung (104),
**dadurch gekennzeichnet, dass** die Durchgangsöffnung (104) an dem distalen Ende (103) des Griffstücks (101) einen ersten Abschnitt (105) aufweist, der einen größeren Innendurchmesser als ein sich an den ersten Abschnitt (105) anschließender zweiter Abschnitt (106) der Durchgangsöffnung (104) hat; und durch
eine innere Hülse (201), die in den zweiten Abschnitt (106) der Durchgangsöffnung (104) des Griffstücks (101) einsetzbar ist und die an einem ersten Ende (202) einen Vorsprung (204) zur Anlage an einer Anlagefläche (107) des Griffstücks (101), die sich am Übergang zwischen dem ersten (105) und dem zweiten Abschnitt (106) der Durchgangsöffnung (104) des Griffstücks (101) befindet, aufweist; und durch
eine äußere Hülse (301), die über einen distalen Abschnitt (114) des Griffstücks (101) am distalen Ende des Griffstücks (101) schiebbar ist;
wobei das Griffstück (101) und die innere Hülse (201) dafür ausgelegt sind, beim Einsetzen der inneren Hülse (201) in den zweiten Abschnitt (106) der Durchgangsöffnung (104) des Griffstücks (101) zwischen dem Vorsprung (204) der inneren Hülse und der Anlagefläche (107) des Griffstücks einen ersten Teil eines Textilstücks (701) zu fixieren; und
wobei das Griffstück (101) und die äußere Hülse (301) dafür ausgelegt sind, beim Schieben der äußeren Hülse (301) über den distalen Abschnitt (114) des Griffstücks (101) zwischen dem distalen Abschnitt (114) des Griffstücks (101) und der äußeren Hülse (301) einen zweiten Teil des Textilstücks (701) zu fixieren.

2. Vorrichtung nach Anspruch 1, die zusätzlich ein Montagewerkzeug (401) umfasst, das an einem ersten Ende (402) einen ersten Abschnitt (404), der in die innere Hülse (201) einsetzbar ist umfasst, sowie einen sich an den ersten Abschnitt (404) anschließenden zweiten Abschnitt (405), der einen größeren Außendurchmesser als der erste Abschnitt (404) hat und in den ersten Abschnitt (105) der Durchgangsöffnung (104) des Griffstücks (101) einsetzbar ist, sowie einen sich an den zweiten Abschnitt (405) anschließenden dritten Abschnitt (406), der einen größeren Außendurchmesser als der zweite Abschnitt (405) hat und in die äußere Hülse (301) einsetzbar ist.

3. Vorrichtung nach Anspruch 2, wobei das Montagewerkzeug (401) an einem zweiten Ende (403) eine Standfläche (407) zum Stellen des Montagewerkzeugs (401) auf eine Fläche aufweist.

4. Vorrichtung nach einem der vorhergehenden Ansprüche, die zusätzlich einen Dorn (501) mit einer Spitze (502) zum Durchstechen des Textilstücks (701) umfasst, wobei der Dorn (501) an einem von der Spitze (502) abgewandten Ende einen Halteabschnitt (503) aufweist, der an dem von ihrem ersten Ende (202) abgewandten zweiten Ende (203) der inneren Hülse (201) in die innere Hülse (201) einsetzbar ist.

5. Vorrichtung nach Anspruch 4, wobei ein Teil des Dorns (501) einen größeren Außendurchmesser als der zweite Abschnitt (106) der Durchgangsöffnung (104) des Griffstücks (101) hat, so dass der Dorn (501) nicht durch den zweiten Abschnitt (106) der Durchgangsöffnung (104) passt.

6. Vorrichtung nach einem der vorhergehenden Ansprüche, wobei die innere Hülse (201) einen oder mehrere, insbesondere drei, Schlitze (205, 206, 207) aufweist.

7. Vorrichtung nach Anspruch 6, wobei die innere Hülse (201) in dem Bereich, in dem die Schlitze (205, 206, 207) angeordnet sind, eine nach außen gewölbte Wand hat, die eine Elastizität aufweist, um die in den zweiten Abschnitt (106) der Durchgangsöffnung (104) des Griffstücks (101) eingesetzte innere Hülse (201) an der Innenwand des zweiten Abschnitts (106) der Durchgangsöffnung (104) des Griffstücks (101) festzuhalten.

8. Vorrichtung nach einem der vorhergehenden Ansprüche, wobei der distale Abschnitt (114) des Griffstücks (101) einen ersten Bereich (108) und einen zweiten Bereich (109) aufweist, die jeweils einen größeren Außendurchmesser als ein zwischen dem ersten (108) und dem zweiten Bereich (109) angeordneter dritter Bereich (110) haben, wobei der erste (108) und der zweite Bereich (109) des Griffstücks (101) an den Enden der äußere Hülse (301) angeordnet sind, wenn die äußere Hülse (301) über den distalen Abschnitt (114) des Griffstücks (101) geschoben ist.

9. Vorrichtung nach Anspruch 8, wobei der distale Abschnitt (114) des Griffstücks (101) in dem dritten Bereich (110) einen oder mehrere, insbesondere zwei, Schlitze (112, 113) aufweist, die gegenüber von dem zweiten Ende (203) der inneren Hülse (201) und/oder dem einen oder den mehreren Schlitzen (205, 206, 207) der inneren Hülse (201) angeordnet sind, wenn die innere Hülse (201) in den zweiten Abschnitt (106) der Durchgangsöffnung (104) des Griffstücks (101) eingesetzt ist.

10. Vorrichtung nach Anspruch 8 oder 9, wobei der zweite Bereich (109) des distalen Abschnitts (114) des Griffstücks (101) weiter vom distalen Ende des Griffstücks (101) entfernt ist als der erste Bereich (108) des distalen Abschnitts (114) des Griffstücks (101), und der Außendurchmesser des zweiten Bereichs (109) des distalen Abschnitts (114) des Griffstücks (101) und ein Innendurchmesser der äußeren Hülse (301) so ausgelegt sind, dass der zweite Bereich (109) des distalen Abschnitts (114) des Griffstücks (101) einen Anschlag für die äußere Hülse (301) bildet, wenn sich das Textilstück (701) zwischen dem zweiten Bereich (109) des distalen Abschnitts (114) des Griffstücks (101) und der äußeren Hülse (301) befindet und die äußere Hülse (301) über den zweiten Bereich (109) des distalen Abschnitts (114) des Griffstücks (101) hinaus schiebbar ist, wenn sich das Textilstück (701) nicht zwischen dem zweiten Bereich (109) des distalen Abschnitts (114) des Griffstücks (101) und der äußeren Hülse (301) befindet.

11. Vorrichtung nach einem der vorhergehenden Ansprüche, wobei das Griffstück (101) an dem proximalen Ende (102) mehrere axial verlaufende Rillen (111) an der Außenseite des Griffstücks (101) aufweist; und/oder
wobei der erste Abschnitt (105) der Durchgangsöffnung (104) an seiner Wandfläche eine Riffelung aufweist.

12. Vorrichtung nach einem der vorhergehenden Ansprüche, wobei die Durchgangsöffnung (104) des Griffstücks (101) am proximalen Ende (102) des Griffstücks (101) einen Innendurchmesser hat, der kleiner als ein Außendurchmesser der inneren Hülse (201) und kleiner als der Innendurchmesser des zweiten Abschnitts (106) der Durchgangsöffnung (104) am distalen Ende (103) des Griffstücks (101) ist.

13. Chirurgisches System, umfassend:
eine Vorrichtung zur Reinigung eines Konus (602) eines Implantats (601) nach einem der vorhergehenden Ansprüche; und
ein Implantat (601), das einen Konus (602) aufweist, wobei der Konus (602) des Implantats (601) und der erste Abschnitt der Durchgangsöffnung (104) des Griffstücks (101) der Vorrichtung zur Reinigung eines Konus (602) eines Implantats (601) zueinander korrespondierende Formen haben.

14. Chirurgisches System nach Anspruch 13 wobei das Implantat (601) ein Verankerungsschaft einer Endoprothese, insbesondere einer Hüft-, Schulter- oder Knieprothese, ist.

15. Chirurgisches System nach Anspruch 13 oder 14, wobei der Konus (602) des Implantats (601) eine Öffnung mit einem Innengewinde aufweist, wobei eine Achse der Öffnung des Konus (602) des Implantats (601) auf einer Kegelachse des Konus (602) liegt, und das System zusätzlich einen Führungsstab (603) umfasst, der in die Öffnung des Konus (602) des Implantats (601) einschraubbar ist, und wobei der Führungsstab (603) durch die Durchgangsöffnung (104) des Griffstücks (101) und die in den zweiten Abschnitt (106) der Durchgangsöffnung (104) des Griffstücks (101) eingesetzte innere Hülse (201) der Vorrichtung zur Reinigung eines Konus (602) einer Endoprothese führbar ist.

## Claims

1. Device for cleaning a cone (602) of an implant (601), comprising:
a handle piece (101) with a proximal end (102), a distal end (103) and a through opening (104) extending axially through the handle piece (101),
**characterized in that**
the through opening (104) has, at the distal end (103) of the handle piece (101), a first section (105) which has a larger inner diameter than a second section (106) of the through opening (104) adjoining the first section (105); and by
an inner sleeve (201) insertable into the second section (106) of the through opening (104) of the handle piece (101) and having, at a first end (202), a protrusion (204) for abutment against an abutment surface (107) of the handle piece (101) located at the transition between the first (105) and the second section (106) of the through opening (104) of the handle piece (101); and by
an outer sleeve (301) slidable over a distal section (114) of the handle piece (101) at the distal end of the handle piece (101);
wherein the handle piece (101) and the inner sleeve (201) are adapted to fix a first part of a textile piece (701) between the protrusion (204) of the inner sleeve and the abutment surface (107) of the handle piece upon insertion of the inner sleeve (201) into the second section (106) of the through opening (104) of the handle piece (101); and
wherein the handle piece (101) and the outer sleeve (301) are adapted to fix a second part of the textile piece (701) between the distal section (114) of the handle piece (101) and the outer sleeve (301) when the outer sleeve (301) is slid over the distal section (114) of the handle piece (101).

2. Device according to claim 1, additionally comprising an assembly tool (401) comprising, at a first end (402), a first section (404) insertable into the inner sleeve (201), and a second section (405) adjoining the first section (404), which has a larger outer diameter than the first section (404) and is insertable into the first section (105) of the through opening (104) of the handle piece (101), and a third section (406) adjoining the second section (405), which has a larger outer diameter than the second section (405) and is insertable into the outer sleeve (301).

3. Device according to claim 2, wherein the assembly tool (401) has a standing surface (407) at a second end (403) for placing the assembly tool (401) on a surface.

4. Device according to one of the preceding claims, which additionally comprises a mandrel (501) with a tip (502) for piercing the textile piece (701), wherein the mandrel (501) has, at an end facing away from the tip (502), a holding section (503) which is insertable into the inner sleeve (201) at the second end (203) of the inner sleeve (201) facing away from its first end (202).

5. Device according to claim 4, wherein a part of the mandrel (501) has a larger outer diameter than the second section (106) of the through opening (104) of the handle piece (101), so that the mandrel (501) does not fit through the second section (106) of the through opening (104).

6. Device according to one of the preceding claims, wherein the inner sleeve (201) has one or more, in particular three, slots (205, 206, 207).

7. Device according to claim 6, wherein the inner sleeve (201) in the region where the slots (205, 206, 207) are arranged has an outwardly curved wall having an elasticity to hold the inner sleeve (201) inserted into the second section (106) of the through opening (104) of the handle piece (101) against the inner wall of the second section (106) of the through opening (104) of the handle piece (101).

8. Device according to one of the preceding claims, wherein the distal section (114) of the handle piece (101) has a first region (108) and a second region (109), each having a larger outer diameter than a third region (110) arranged between the first (108) and the second region (109), wherein the first (108) and second regions (109) of the handle piece (101) are arranged at the ends of the outer sleeve (301) when the outer sleeve (301) is slid over the distal section (114) of the handle piece (101).

9. Device according to claim 8, wherein the distal section (114) of the handle piece (101) in the third region (110) has one or more, in particular two, slots (112, 113) arranged opposite the second end (203) of the inner sleeve (201) and/or the one or more slots (205, 206, 207) of the inner sleeve (201) when the inner sleeve (201) is inserted into the second section (106) of the through opening (104) of the handle piece (101).

10. Device according to claim 8 or9, wherein the second region (109) of the distal section (114) of the handle piece (101) is further away from the distal end of the handle piece (101) than the first region (108) of the distal section (114) of the handle piece (101), and the outer diameter of the second region (109) of the distal section (114) of the handle piece (101) and an inner diameter of the outer sleeve (301) are configured such that the second region (109) of the distal section (114) of the handle piece (101) forms a limit stop for the outer sleeve (301), when the textile piece (701) is located between the second region (109) of the distal section (114) of the handle piece (101) and the outer sleeve (301), and the outer sleeve (301) is slidable beyond the second region (109) of the distal section (114) of the handle piece (101) when the textile piece (701) is not located between the second region (109) of the distal section (114) of the handle piece (101) and the outer sleeve (301).

11. Device according to one of the preceding claims, wherein the handle piece (101) at the proximal end (102) has a plurality of axially extending grooves (111) on the outer side of the handle piece (101); and/or
wherein the first section (105) of the through opening (104) has a corrugation on its wall surface.

12. Device according to one of the preceding claims, wherein the through opening (104) of the handle piece (101) at the proximal end (102) of the handle piece (101) has an inner diameter that is smaller than an outer diameter of the inner sleeve (201) and smaller than the inner diameter of the second section (106) of the through opening (104) at the distal end (103) of the handle piece (101).

13. Surgical system comprising:
a device for cleaning a cone (602) of an implant (601) according to one of the preceding claims; and
an implant (601) having a cone (602), wherein the cone (602) of the implant (601) and the first section of the through opening (104) of the handle piece (101) of the device for cleaning a cone (602) of an implant (601) have corresponding shapes.

14. Surgical system according to claim 13, wherein the implant (601) is an anchoring stem of an endoprosthesis, in particular a hip, shoulder or knee prosthesis.

15. Surgical system according to claim 13 or 14, wherein the cone (602) of the implant (601) has an opening with an inner thread, wherein an axis of the opening of the cone (602) of the implant (601) lies on a cone axis of the cone (602), and the system additionally comprises a guide rod (603), which can be screwed into the opening of the cone (602) of the implant (601), and wherein the guide rod (603) can be guided through the through opening (104) of the handle piece (101) and the inner sleeve (201) of the device for cleaning a cone (602) of an endoprosthesis that is inserted into the second section (106) of the through opening (104) of the handle piece (101).

## Revendications

1. Dispositif destiné au nettoyage d'un cône (602) d'un implant (601), comprenant :
une poignée (101) comportant une extrémité proximale (102), une extrémité distale (103) et une ouverture traversante (104) s'étendant axialement à travers la poignée (101),
**caractérisé en ce que** l'ouverture traversante (104) présente, au niveau de l'extrémité distale (103) de la poignée (101), une première section (105) qui a un diamètre intérieur plus grand qu'une deuxième section (106) de l'ouverture traversante (104) adjacente à la première section (105) ; et par
un manchon intérieur (201) pouvant être inséré dans la deuxième partie (106) de l'ouverture traversante (104) de la poignée (101) et comportant, à une première extrémité (202) une saillie (204) destinée à venir en appui contre une surface d'appui (107) de la poignée (101), située à la jonction entre la première (105) et la deuxième partie (106) de l'ouverture traversante (104) de la poignée (101) ; et par
un manchon extérieur (301) pouvant être glissé sur une partie distale (114) de la poignée (101) à l'extrémité distale de la poignée (101) ;
la poignée (101) et le manchon intérieur (201) étant conçus pour, lors de l'insertion du manchon intérieur (201) dans la deuxième partie (106) de l'ouverture traversante (104) de la poignée (101), fixer une première partie d'un morceau de textile (701) entre la saillie (204) du manchon intérieur et la surface d'appui (107) de la poignée ; et
la poignée (101) et le manchon extérieur (301) étant conçus pour, lors de l'enfoncement du manchon extérieur (301) sur la partie distale (114) de la poignée (101), fixer une deuxième partie du morceau de textile (701) entre la partie distale (114) de la poignée (101) et le manchon extérieur (301).

2. Dispositif selon la revendication 1, comprenant en outre un outil de montage (401) qui comporte, à une première extrémité (402), une première partie (404) pouvant être insérée dans le manchon intérieur (201), ainsi qu'une deuxième partie (405) se raccordant à la première partie (404), qui présente un diamètre extérieur supérieur à celui de la première partie (404) et qui peut être insérée dans la première partie (105) de l'ouverture traversante (104) de la poignée (101), ainsi qu'une troisième partie (406) se raccordant à la deuxième partie (405), qui présente un diamètre extérieur supérieur à celui de la deuxième partie (405) et qui peut être insérée dans le manchon extérieur (301).

3. Dispositif selon la revendication 2, dans lequel l'outil de montage (401) comporte, à une deuxième extrémité (403), une surface d'appui (407) permettant de poser l'outil de montage (401) sur une surface.

4. Dispositif selon l'une des revendications précédentes, comprenant en outre un mandrin (501) muni d'une pointe (502) destinée à percer le morceau de textile (701), le mandrin (501) comportant, à une extrémité opposée à la pointe (502), une partie de retenue (503) qui peut être insérée dans le manchon intérieur (201) au niveau de la deuxième extrémité (203) de celle-ci, opposée à sa première extrémité (202) du manchon intérieur (201.

5. Dispositif selon la revendication 4, dans lequel une partie du mandrin (501) présente un diamètre extérieur supérieur à celui de la deuxième partie (106) de l'ouverture traversante (104) de la poignée (101), de sorte que le mandrin (501) ne passe pas à travers la deuxième partie (106) de l'ouverture traversante (104).

6. Dispositif selon l'une des revendications précédentes, dans lequel le manchon intérieur (201) comporte une ou plusieurs fentes (205, 206, 207), en particulier trois.

7. Dispositif selon la revendication 6, dans lequel le manchon intérieur (201) présente, dans la zone où sont disposées les fentes (205, 206, 207), une paroi bombée vers l'extérieur qui présente une élasticité permettant au manchon intérieur (201) inséré dans la deuxième partie (106) de l'ouverture traversante (104) de la poignée (101) de s'accrocher à la paroi intérieure de la deuxième partie (106) de l'ouverture de passage (104) de la poignée (101).

8. Dispositif selon l'une des revendications précédentes, dans lequel la partie distale (114) de la poignée (101) comporte une première zone (108) et une deuxième zone (109) qui présentent chacune un diamètre extérieur supérieur à celui d'une troisième zone (110) située entre la première (108) et la deuxième zone (109) (110), la première (108) et la deuxième zone (109) de la poignée (101) étant disposées aux extrémités du manchon extérieur (301) lorsque le manchon extérieur (301) est enfilé sur la partie distale (114) de la poignée (101).

9. Dispositif selon la revendication 8, dans lequel la partie distale (114) de la poignée (101) comporte, dans la troisième zone (110), une ou plusieurs fentes (112, 113), en particulier deux, qui sont disposées en face de la deuxième extrémité (203) du manchon intérieur (201) et/ou de la ou des fentes (205, 206, 207) du manchon intérieur (201) lorsque le manchon intérieur (201) est inséré dans la deuxième partie (106) de l'ouverture traversante (104) de la poignée (101).

10. Dispositif selon la revendication 8 ou 9, dans lequel la deuxième zone (109) de la partie distale (114) de la poignée (101) est plus éloignée de l'extrémité distale de la poignée (101) que la première zone (108) de la partie distale (114) de la poignée (101), et le diamètre extérieur de la deuxième zone (109) de la partie distale (114) de la poignée (101) et un diamètre intérieur du manchon extérieur (301) sont conçus de telle sorte que la deuxième zone (109) de la partie distale (114) de la poignée (101) forme une butée pour le manchon extérieur (301) lorsque le morceau de textile (701) se trouve entre la deuxième zone (109) de la partie distale (114) de la poignée (101) et le manchon extérieur (301) et que le manchon extérieur (301) peut être poussé au-delà de la deuxième zone (109) de la partie distale (114) de la poignée (101) lorsque le morceau de textile (701) ne se trouve pas entre la deuxième zone (109) de la partie distale (114) de la poignée (101) et le manchon extérieur (301).

11. Dispositif selon l'une des revendications précédentes, dans lequel la poignée (101) présente, à son extrémité proximale (102), plusieurs rainures (111) s'étendant axialement sur la face extérieure de la poignée (101) ; et/ou
dans lequel la première partie (105) de l'ouverture traversante (104) présente une cannelure sur sa surface de paroi.

12. Dispositif selon l'une des revendications précédentes, dans lequel l'ouverture traversante (104) de la poignée (101) à l'extrémité proximale (102) de la poignée (101) a un diamètre intérieur qui est inférieur au diamètre extérieur du manchon intérieur (201) et inférieur au diamètre intérieur de la deuxième partie (106) de l'ouverture traversante (104) à l'extrémité distale (103) de la poignée (101).

13. Système chirurgical comprenant :
un dispositif destiné à nettoyer un cône (602) d'un implant (601) selon l'une des revendications précédentes ; et
un implant (601) comportant un cône (602), le cône (602) de l'implant (601) et la première partie de l'ouverture traversante (104) de la poignée (101) du dispositif de nettoyage d'un cône (602) d'un implant (601) ayant des formes correspondant l'une à l'autre.

14. Système chirurgical selon la revendication 13, dans lequel l'implant (601) est une tige d'ancrage d'une endoprothèse, en particulier d'une prothèse de hanche, d'épaule ou de genou.

15. Système chirurgical selon la revendication 13 ou 14, dans lequel le cône (602) de l'implant (601) présente une ouverture à filetage intérieur, un axe de l'ouverture du cône (602) de l'implant (601) coïncidant avec un axe conique du cône (602), et le système comprend en outre une tige de guidage (603) pouvant être vissée dans l'ouverture du cône (602) de l'implant (601), et la tige de guidage (603) pouvant guidée à travers l'ouverture traversante (104) de la poignée (101) et le manchon intérieur (201) du dispositif de nettoyage d'un cône (602) d'une endoprothèse, insérée dans la deuxième partie (106) de l'ouverture traversante (104) de la poignée (101).
